# EUROPEAN PATENT APPLICATION

(11) **EP 4 265 260 A1**
(43) Date of publication of application: **25.10.2023**
(21) Application number: 21909327.5
(22) Date of filing: 20.12.2021
(51) Int. Cl.: A61K 31/7048, C07J 71/00, A61K 9/08, A61K 9/10, A61K 9/12

(54) **STABLE LIQUID PHARMACEUTICAL COMPOSITION CONTAINING KUDING SAPONIN COMPOUND**

(30) Priority: 21.12.2020 CN 202011517217
(71) Applicant: Shanghai KE Pharmaceutical Co., Ltd, Shanghai 200023 (CN)
(72) Inventor: MA, Ming, Shanghai 200023 (CN)
(74) Representative: Pace Napoleone, Maria
(86) International application number: PCT/CN2021/139611
(87) International publication number: WO 2022/135331

(57) **Abstract**

A stable liquid pharmaceutical composition containing a Kuding saponin compound, containing a therapeutically effective amount of Kuding saponin compound, a buffer effective amount of buffer solution having a pH of 6.5-7.5, and a pharmaceutically acceptable carrier. The liquid pharmaceutical composition is suitable for being stored in a semi-permeable container for long-term storage. The semi-permeable container is preferably a combination of a low-density polyethylene bottle and a medicinal aluminum foil bag, and a vacuum state or approximately vacuum state exists between the low-density polyethylene bottle and the medicinal aluminum foil bag.

## Description

### TECHNICAL FIELD

The present invention belongs to the field of chemical pharmaceutical area, and in particular, it relates to a stable liquid pharmaceutical composition comprising Kuding saponin compounds.

### BACKGROUND

Chronic obstructive pulmonary disease (COPD) is chronic bronchitis or emphysema with airflow blocking characteristics, which can be further developed into pulmonary heart disease and respiratory failure. The characteristic pathological changes of COPD include chronic inflammation of small airway and lung caused by pathogenic bacteria and gas exposure, pulmonary parenchyma damage and small airway fibrosis caused by the comprehensive effect of mechanisms such as oxidative stress and protease increase, and etc. The morbidity and mortality of COPD continuously rise along with the changes in the global environment and the world population age structure, and the morbidity of the population over 40 years old worldwide reaches 9% -10%, causing serious impacts on patients' life and work.

At present, COPD is mainly treated by medicament(s), which could effectively control the development of disease and relieve symptoms. The therapeutic medicaments for COPD mainly comprise bronchodilators (β2-adrenergic receptor agonists, theophylline drugs and anticholinergic drugs), glucocorticoids, antibacterials, and mucus-active agents. As the further understanding of the pathogenesis and pathological features of COPD, a variety of new medicaments for treating COPD, including anti-protease formulations, phosphodiesterase inhibitors, P38 mitogen-activated protein inhibitors, and phosphoinositide -3 kinase inhibitors, appear.

Bronchial asthma (referred to as "asthma") is a disease characterized by chronic allergic respiratory tract inflammation, and many cells and cytokines participate in this process. Asthma would cause high reactivity of the airway, leading to the repeated onset of stridor, dyspnea, and coughing, and its sustained onset may lead to death. Asthma often occurs at night and in the early morning, and often relates to the contact with allergens such as environment, air flow, dust mites, pollen, and etc., as well as autoimmunity. At present, there are about 300,000,000 people suffering from asthma worldwide, with about 20,000,000 people suffering from said illness in China, and the incidence of asthma shows an ascendant trend. The high incidence of asthma affects people's physical and mental health and quality of life, and therefore, it has become one of the major chronic diseases seriously threatening public health.

At present, there are two major categories of successfully developed medicaments for treating asthma worldwide, that is, respiratory smooth muscle relaxants and anti-inflammatory agents, which mainly includ β-receptor agonists, glucocorticoids, aminophylline, and the like. Although the short-term efficacies of these medicaments are significant, the effects on increasing the survival rate and improving the life quality are not significant. The main reason is that these medicaments have substantial side effects and would cause certain damages to heart and kidney functions. In order to solve these problems, new forms have been developed continuously, and it is desirable to obtain anti-asthma medicaments with lower toxicity and better therapeutical effect.

Kudingcha is a plant tea that is often drunk by people in Southern China, and it is also a traditional medicinal plant. Kudingcha is the general name for a large class of plants with different degrees of bittersweet taste that derived from plant leaves such asherbs, liana, shrubs, small trees, tall trees, and the like. The native plants of Kudingcha relate to 12 families and more than 30 species, and among them are two families that are currently planted in a large area and widely used: the first one is Kudingcha from the plant of genus *Ilex* in family *Aquifoliaceae* mainly comprising *Ilex kudingcha C.J Tseng, Ilex latifolia Thunb., Ilex cornute Lindl., Ilex. Pentag-ona S.K.Chen. Y.X.Feng et C.F.Liang, Ilex centro-chinensis S.Y.Hu,* and *I. houshanensis Y.H.He;* and the second one is Kudingcha from the family *Oleaceae,* i.e, Kudingcha from the plant of genus *Ligustrum,* mainly developed in the provinces and cities of South-western China such as Guizhou, Sichuan, Yunnan, and Chongqing; and Kudingcha from the plant of this genus is being called "small leaf Kudingcha" (*Ligustrum henryi Hemsl*)*.*

Modern scientific researches have proved that the leaves of Kudingcha comprise caffeine, tannic acid, protein, ursolic acid, aromatic oil, kudinosides, α-balsam, β-sitosterol, ursolic acid and various vitamins and trace elements, and have medicinal health-care functions such as anti-fever and detoxification, anti-inflammation, sterilizing, cough-relieving and phlegm- reducing, stomach-invigorating and digest-improving, eyesight-improving, mentality- improving, cholesterol-reducing, antihypersensitive, antihyperlipidemic, anti-fatigue, anti-aging functions. The Chinese patent CN106456657A discloses the use of Kuding saponin compounds in the treatment of lung diseases such as asthma and COPD. These compounds can be administered in an inhalation manner and have the advantages of rapid onset, large lung contact surface area, and the like.

Obtaining stable chemical formulations of these compounds is an urgent problem to be solved in the pharmaceutical industry.

### SUMMARY OF THE INVENTION

The present invention provides a stable liquid pharmaceutical composition, characterized in that the liquid pharmaceutical composition comprises a therapeutically effective amount of a Kuding saponin compound, a buffering effective amount of a buffer solution having a pH of 6.5-7.5, and a pharmaceutically acceptable carrier.

In a preferred embodiment, the Kuding saponin compound is separated from a plant of genus *Ilex* in family *Aquifoliaceae*; more preferably, the plant of genus *Ilex* in family Aquifoliaceae is selected from the group consisting of *Ilex kudingcha C.J Tseng, Ilex latifolia Thunb., Ilex cornute Lindl., Ilex. Pentagona S.K.Chen. Y.X.Feng et C.F.Liang, Ilex centro-chinensis S.Y.Hu* or *Ilex houshanensis Y.H.He.*

In another preferred embodiment, the Kuding saponin compound is a compound of formula (I): 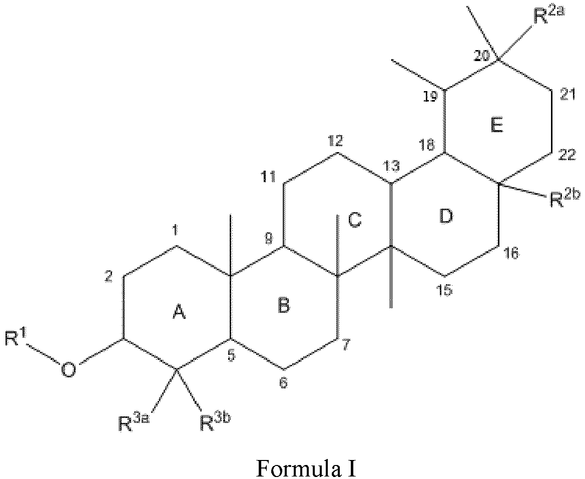 wherein:
ring A, ring B, ring C, ring D, or ring E are each independently a fully saturated or partially saturated ring;
positions C2, C11, C12, and C19 are independently optionally substituted by-OH, respectively;
R¹ is selected from a sugar residue;
R^{2a} and R^{2b} together form -CO₂-; R^{3a} and R^{3b} together form CH₂=, or are independently selected from -CH₃ or -CH₂-OH, respectively.

In a preferred embodiment, the sugar residue is a monosaccharide residues or an oligosaccharide residues. In a preferred embodiment, the monosaccharide is arabinose (Ara), glucuronic acid or 2-deoxy-glucuronic acid (GlcA), glucose (Glc), or rhamnose (Rha). In some embodiments, the oligosaccharide residue is a disaccharide residue, a trisaccharide residue, ora tetrasaccharide residue. In some embodiments, the oligosaccharide residue comprises any combination of glucose, arabinose, glucuronic acid, and rhamnose.

In a preferred embodiment, the ring A, ring B, ring C, and ring E are fully saturated rings; the ring D is a partially saturated ring; positions C12 and C19 are independently substituted by - OH, respectively; R¹ is a monosaccharide residue or an oligosaccharide residue; and R^{3a} and R^{3b} are -CH₃, respectively.

In a preferred embodiment, the ring A, ring B, ring C, and ring E are fully saturated rings; ring D is a partially saturated ring; positions C11 and C19 are independently substituted by -OH, respectively; R^{3a} and R^{3b} are -CH₃, respectively, and R¹ is a monosaccharide residue or an oligosaccharide residue.

In a preferred embodiment, ring A, ring B, and ring E are fully saturated rings; ring C and ring D are partially saturated rings; position C19 is substituted by -OH; R^{3a} and R^{3b} are -CH₃, respectively; and R¹ is a monosaccharide residue or an oligosaccharide residue.

In a preferred embodiment, the compound of formula (I) has a structure of following formula (II), (III), or (IV): 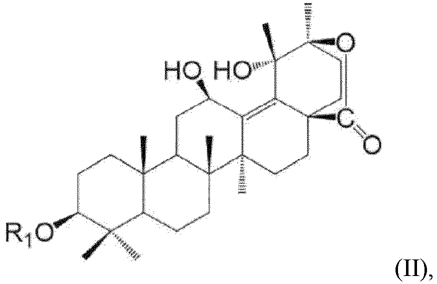 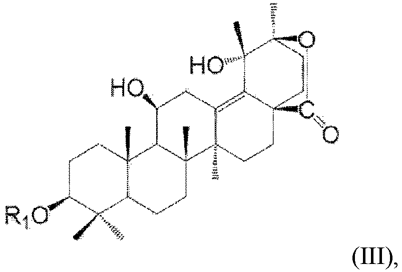 or 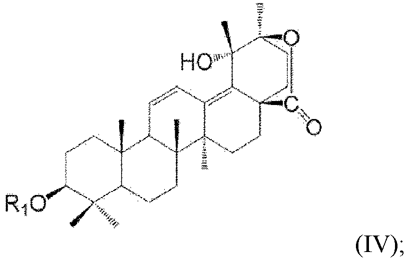 wherein in each formula, R₁ is a monosaccharide residue or an oligosaccharide residue. In a preferred embodiment, in formulae (II), (III), and (IV), the monosaccharide is arabinose (Ara), glucuronic acid, 2-deoxy-glucuronic acid (GlcA), glucose (Glc) or rhamnose (Rha); the oligosaccharide residue is a disaccharide residue, a trisaccharide residue, or a tetrasaccharide residue; and the oligosaccharide residue comprises any combination of glucose, arabinose, and rhamnose.

In a preferred embodiment, the Kuding saponin compound is selected from the group consisting of Kudinoside A, Kudinoside B, Kudinoside C, Kudinoside D, Kudinoside E, Kudinoside F, Kudinoside I, Kudinoside J, Ilekudinoside H, Ilekudinoside I, and Ilekudinoside J.

In a preferred embodiment, the Kuding saponin compound is selected from the group consisting of Kudinoside A, Kudinoside B, Kudinoside C, Kudinoside D, Kudinoside I, Ilekudinoside I, and Ilekudinoside J.

In a preferred embodiment, the Kuding saponin compound is one of the two isomers of Kudinoside A: 3β-12α-19α-trihydroxy-ursane-13(18)-ene-28, 20β-lactone-3-O-[β-D-glucosyl-(1→3)-[α-L-rhamnosyl-(1→2)]-α-L-arabinoside, and/or 3β-12β-19α-trihydroxy-ursane-13(18)-ene-28,20β-lactone-3-O-[β-D-glucosyl-(1→3)-[α-L-rhamnosyl-(1→ 2) ]-α-L-arabinoside.

In a most preferred embodiment, the mass percentage of Kuding saponin compound in the whole liquid pharmaceutical composition is 0.001-0.050%, preferably 0.006-0.030%, more preferably 0.010-0.030%, and more preferably 0.015%.

In another preferred embodiment, the buffer solution having a pH of 6.5-7.5 is a phosphate buffer, preferably a sodium phosphate buffer, a potassium phosphate buffer, or a combination thereof, more preferably a disodium hydrogen phosphate-potassium dihydrogen phosphate buffer.

In a more preferred embodiment, the buffer solution having a pH of 6.5-7.5 is selected from a disodium hydrogen phosphate-potassium dihydrogen phosphate buffer having a pH of 6.5, a disodium hydrogen phosphate-potassium dihydrogen phosphate buffer having a pH of 6.9, a disodium hydrogen phosphate-potassium dihydrogen phosphate buffer having a pH of 7.0, a disodium hydrogen phosphate-potassium dihydrogen phosphate buffer having a pH of 7.4, or a disodium hydrogen phosphate-potassium dihydrogen phosphate buffer having a pH of 7.5.

In a preferred embodiment, the pharmaceutically acceptable carrier comprises a pharmaceutically acceptable solvent, a cosolvent, a stabilizer, or a combination thereof.

In a preferred embodiment, the pharmaceutically acceptable solvent is selected from water, ethanol, or a combination thereof; preferably an ethanol aqueous solution.

In a more preferred embodiment, among the pharmaceutically acceptable solvent, the mass percentage of ethanol in the whole liquid pharmaceutical composition is 1.0-5.0%; more preferably 2.5-4.0%, even more preferably 3.5%.

In another preferred embodiment, the cosolvent is selected from the group consisting of propanediol, glycerol, polyethylene glycol 200, polyethylene glycol 400 or Tween 80; preferably Tween 80.

In another more preferred embodiment, the mass percentage of the cosolvent in the whole liquid pharmaceutical composition is 0.1% -0.8%, preferably 0.2% -0.5%.

In yet another preferred embodiment, the cosolvent is Tween 80, and the mass percentage of Tween 80 in the whole liquid pharmaceutical composition is 0.1% -0.5%, preferably 0.2% - 0.4%, and more preferably 0.3%.

In yet another preferred embodiment, the stabilizer is selected from the group consisting of phenylethanol or disodium edetate, preferably disodium edetate.

In a preferred embodiment, the mass percentage of the stabilizer in the whole liquid pharmaceutical composition is 0.05%-0.20%, preferably 0.05% -0.15%.

In a preferred embodiment, the mass percentage of disodium edetate in the whole liquid pharmaceutical composition is 0.05%-0.20%; preferably 0.05%-0.10%, and more preferably 0.072% -0.088%.

In a preferred embodiment, the buffer system of the liquid pharmaceutical composition is disodium hydrogen phosphate-potassium dihydrogen phosphate, and the pH of the composition is about 7.0; wherein the liquid pharmaceutical composition comprises, based on the whole weight of the liquid pharmaceutical composition: 0.006% -0.030% of Kudinoside A; about 3.5% ethanol; 0.1% -0.5% of Tween 80; and 0.072% -0.088% of disodium edetate.

In a preferred embodiment, the liquid pharmaceutical composition is characterized in that the liquid pharmaceutical composition comprises, based on the total weight of the liquid pharmaceutical composition,
about 0.006% of Kudinoside A, about 3.5% of ethanol, about 0.1% of Tween 80, about 0.072% of disodium edetate, and about 96.2% of water; or
about 0.015% of Kudinoside A, about 3.5% ethanol, about 0.3% Tween 80, about 0.08% disodium edetate, and about 95.8% water; or
about 0.03% Kudinoside A, about 3.5% ethanol, about 0.5% Tween 80, about 0.088% disodium edetate, and about 95.2% water.

In a preferred embodiment, the liquid pharmaceutical composition is a solution or a suspension.

In a more preferred embodiment, the dosage form of the liquid pharmaceutical composition is an inhalation solution or an inhalation suspension.

In a preferred embodiment, the liquid pharmaceutical composition is stored in a sealed pharmaceutically acceptable packaging material.

In a more preferred embodiment, the pharmaceutically acceptable packaging material comprises a polymer material, a glass bottle, an aluminum foil material, or a combination thereof.

In a more preferred embodiment, the polymer material is selected from the group consisting of a low-density polyethylene film, a low-density polyethylene bag, a high-density polyethylene film, a low-density polyethylene bottle, a high-density polyethylene bottle, a polypropylene bottle, a polyethylene terephthalate bottle, a polyester/aluminum/polyethylene composite film, a polyester/aluminum/polyethylene composite bag, or a combination thereof.

In a preferred embodiment, the aluminum foil material comprises an aluminum foil bag, preferably a medicinal aluminum foil bag.

In a more preferred embodiment, the pharmaceutically acceptable packaging material is a combination of a low-density polyethylene bottle and an aluminum foil bag.

In a further preferred embodiment, a vacuum or near vacuum state exists between the low-density polyethylene bottle and the aluminum foil bag.

In a most preferred embodiment, the low-density polyethylene bottle has a thickness of 0.5-2.0 mm, preferably 0.8-1.2 mm, more preferably 1.0-1.2 mm.

In the preparation process of the liquid pharmaceutical composition of the present invention, due to the poor solubility of the Kuding saponin compounds (for example, the Kudinoside A), the preparation process needs to be continuously adjusted so that the compounds can be fully dissolved therein.

In a preferred embodiment, the preparation process of the liquid pharmaceutical composition comprises: weighing water (such as purified water or water for injection use), adding disodium hydrogen phosphate, potassium dihydrogen phosphate, and disodium edetate, stirring uniformly, and adjusting the pH to prepare a buffer solution; then weighing ethanol and Tween 80, adding the solution mentioned above and stirring uniformly; and finally, adding Kuding saponin compounds (such as Kudinoside A) thereinto, and stirring until the complete dissolution.

In another preferred embodiment, the preparation process of the liquid pharmaceutical composition comprises: weighing water (such as purified water or water for injection use), adding disodium hydrogen phosphate, potassium dihydrogen phosphate, and disodium edetate, stirring uniformly, and adjusting the pH to prepare a buffer solution; weighing ethanol and Tween 80, adding a part of the prepared buffer solution thereinto, stirring uniformly to prepare a solution with an ethanol concentration of about 50%, adding Kuding saponin compounds (such as Kudinoside A) thereinto, and stirring until the complete dissolution; and finally, adding the rest of the buffer solution, and stirring uniformly.

In another preferred embodiment, the preparation process of the liquid pharmaceutical composition comprises: weighing water (such as purified water or water for injection use), adding disodium hydrogen phosphate, potassium dihydrogen phosphate, and disodium edetate, stirring uniformly, and adjusting the pH to prepare a buffer solution; weighing ethanol and Tween 80, adding a part of the prepared buffer solution thereinto, stirring uniformly to prepare a solution with an ethanol concentration of about 30%, adding Kuding saponin compounds (such as Kudinoside A) thereinto, and stirring until the complete dissolution; and finally, adding the rest of the buffer solution, and stirring uniformly.

In yet another preferred embodiment, the preparation process of the liquid pharmaceutical composition comprises: weighing water (such as purified water or water for injection use), adding disodium hydrogen phosphate, potassium dihydrogen phosphate and disodium edetate, adjusting the pH, and stirring uniformly to prepare a buffer solution; weighing ethanol and Tween 80, adding Kuding saponin compounds (such as Kudinoside A) thereinto, and stirring until the complete dissolutionto prepare a mother liquor; and finally, mixing the mother liquor with the buffer solution, and stirring uniformly.

### BRIEF DESCRIPTION OF THE DRAWINGS

FIG. 1A: Change in Ethanol Content (%) of Samples 1-3 in Long Term Test (Temperature: 25°C ± 2° C; Humidity: 40% ± 5%).
FIG. 1B: Change in Ethanol Content (%) of Samples 4-6 in Long Term Test (Temperature: 25°C ± 2° C; Humidity: 40% ± 5%).
FIG. 1C: Change in Ethanol Content (%) of Samples 7-9 in Long Term Test (Temperature: 25°C ± 2° C; Humidity: 40% ± 5%).
FIG. 2A: Change in Ethanol Content (%) of Samples 1-3 in Acceleration Test (Temperature: 40°C ± 2° C; Humidity: 22% ± 5%).
FIG. 2B: Change in Ethanol Content (%) of Samples 4-6 in Acceleration Test (Temperature: 40°C ± 2° C; Humidity: 22% ± 5%).
FIG. 2C: Change in Ethanol Content (%) of Samples 7-9 in Acceleration Test (Temperature: 40°C ± 2° C; Humidity: 22% ± 5%).
FIG. 3A: Change in ethanol content (%) of Samples 1-3 in High temperature test (temperature: 60°C ± 2° C.).
FIG. 3B: Change in ethanol content (%) of Samples 4-6 in High temperature test (temperature: 60°C ± 2° C.).
FIG. 3C: Change in ethanol content (%) of Samples 7-9 in High temperature test (temperature: 60°C ± 2° C.).
FIG. 4 shows the structures of the Kuding saponin compounds, wherein FIG. 4A represents kudinoside A, kudinoside B, kudinoside C, kudinoside I, Ilekudinoside I, and Ilekudinoside J; FIG. 4B represents kudinoside F and Ilekudinoside H; and FIG. 4C represents kudinoside D, kudinoside E, and kudinoside J.

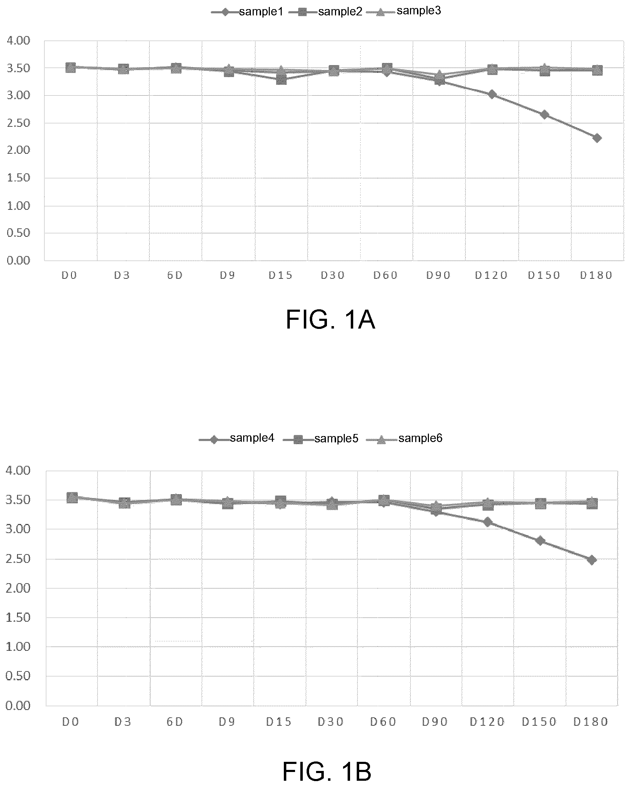

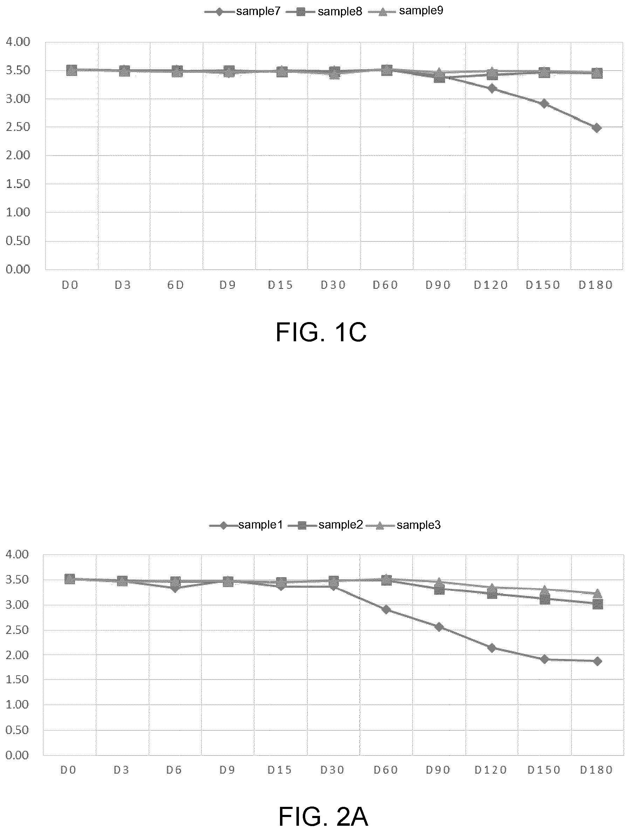

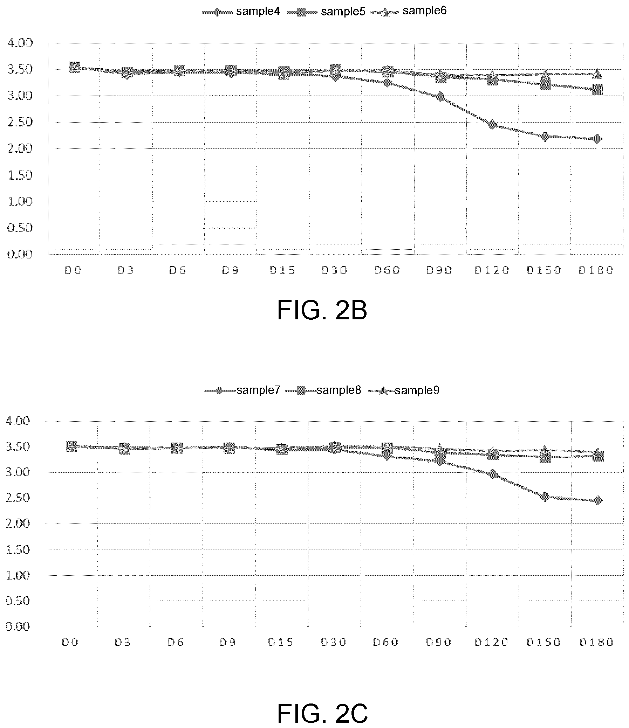

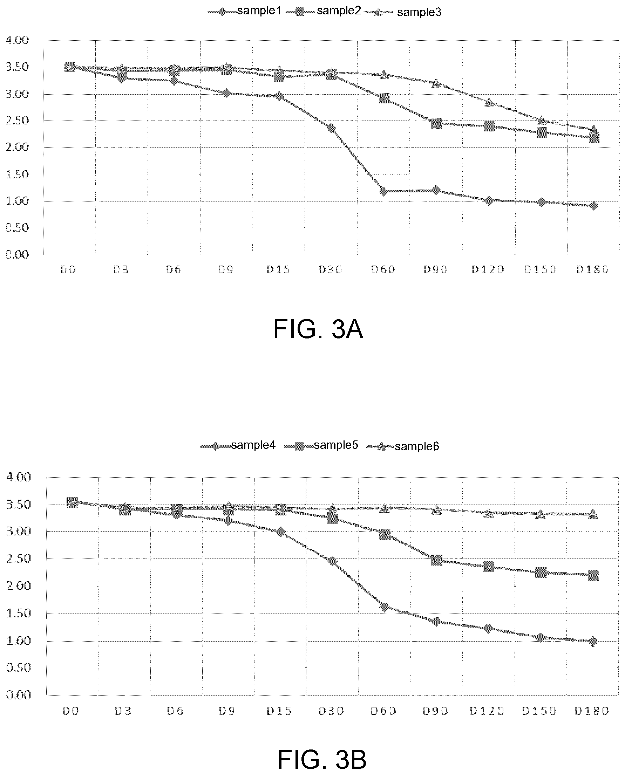

In FIGS. 1A-1C, 2A-2C, and 3A-3C, the abscissa represents the time for detecting the ethanol content, and the ordinate represents the mass percentage of ethanol in the entire liquid pharmaceutical composition.

### DETAILED DESCRIPTION OF THE PREFERRED EMBODIMENTS

In the present specification, the related components or the preferred components thereof can be combined with each other to form a new technical solution, unless the context clearly indicates otherwise.

In the present specification, all embodiments and preferred embodiments mentioned herein can be combined with each other to form a new technical solution, unless the context clearly indicates otherwise.

In the present specification, all the technical features and preferred features mentioned herein can be combined with each other to form a new technical solution, unless the context clearly indicates otherwise.

As used herein, the term "an", "a" and "the" are intended to mean "at least one", unless the context clearly indicates otherwise.

The "range" disclosed herein is in the form of a lower limit and an upper limit. There may be one or more lower limits, and one or more upper limits, respectively. A given range is defined by selecting a lower limit and an upper limit. The selected lower limit and upper limit define the boundaries of a particular range. All ranges able to be defined in this manner are inclusive and combinable, that is, any lower limit may be combined with any upper limit to form a range. As used herein, the range defined by "about" is typically the range of experimental errors. For example, if the experimental error is 0.1, then "about 7" refers to 7 ± 0.1.

In particular, the present invention provides a stable liquid pharmaceutical composition, characterized in that it comprises a therapeutically effective amount of a Kuding saponin compound, a buffering effective amount of a buffer solution having a pH of 6.5-7.5, and a pharmaceutically acceptable carrier. As used herein, the term "comprising", "comprises", "comprise", "including", "includes" , "contains" , "contain", "having", "has", and "with" refer to that the medicament(s) may also contain any other components, and these components may be present in any content/amount, as long as the component present in this content/amount is acceptable to the human body, and there is no substantial influence on the biological activity of the active ingredient in the pharmaceutical composition of the present invention.

In a preferred embodiment, the liquid pharmaceutical composition comprises a therapeutically effective amount of a Kuding saponin compound, a buffering effective amount of a buffer solution having a pH of 6.5-7.5, a cosolvent, a stabilizer, and a pharmaceutically acceptable solvent.

The Kuding saponin compounds of the present invention are in the form of liquid or solid, which are commercially available or can be obtained by separation or chemical synthesis methods in prior art from a plant of genus *Ilex* in family *Aquifoliaceae.* The plant of genus *Ilex* in family *Aquifoliaceae* is selected from the group consisting of *Ilex kudingcha C.J. Tseng, Ilex latifolia Thunb., Ilex cornute Lindl., Ilex. Pentagona S.K.Chen. Y.X.Feng et C.F.Liang, Ilex centro-chinensis S.Y.Hu, I. houshanensis Y.H.He,* or a combination thereof.

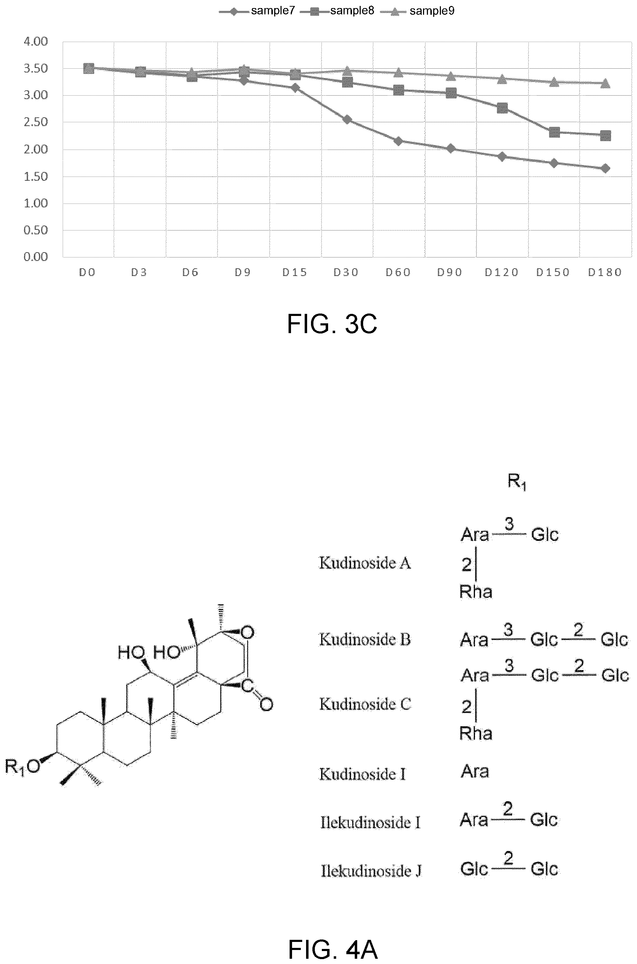

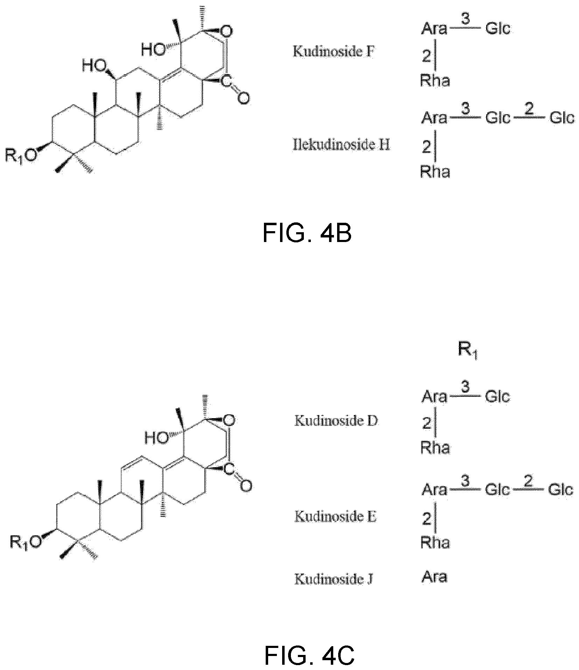

In a preferred embodiment, the Kuding saponin compound is preferably Kudinoside A, Kudinoside B, Kudinoside C, Kudinoside D, Kudinoside E, Kudinoside F, Kudinoside I, Kudinoside J, Ilekudinoside H, Ilekudinoside I, and Ilekudinoside J (the chemical structural formulae thereof are described in CN106456657A and "Triterpenes and saponins thereof from Kudingcha of Genus *Ilex*", particularly see FIGs. 4A-C); more preferably 3β-12α-19α-trihydroxy-ursane-13(18)-ene-28, 20β-lactone-3-O-[β-D-glucosyl-(1→3)-[α-L-rhamnosyl-(1→2)]-α-L-arabinoside and/or 3β-12β-19α-trihydroxy-ursane-13(18)-ene-28,20β-lactone-3-O-[β-D-glucosyl-(1→3)-[α-L-rhamnosyl-(1→2) ]-α-L-arabinoside. The term "therapeutically effective amount" refers to the concentration (dose) when the liquid pharmaceutical composition of the present invention is used to the treatment of diseases (eg, asthma, COPD, or other related diseases). In general, the mass percentage of the "therapeutically effective amount" of the Kuding saponin compounds in the whole liquid pharmaceutical composition is 0.001% -0.050%, such as 0.006-0.030%. In some embodiments, the mass percentage of Kuding saponin compounds in the whole liquid pharmaceutical composition is 0.001-0.050%, preferably 0.006-0.030%, more preferably 0.010-0.030%, and even more preferably 0.015%.

The term "pharmaceutically acceptable carrier" refers to a substance that does not substantially reduce the therapeutical effect of a medicament after it is blending with the Kuding saponin compound in the medicament of the present invention, including but not limited to an adjuvant, an excipient, a cosolvent, a sweetener, a diluent, a preservative, a dye/colorant, a flavoring agent, a surfactant, a wetting agent, a dispersant, a suspending agent, a stabilizer, an isotonic, a solvent, or an emulsifier that its use has been approved by National Medical Products Administration (NMPA). In some embodiments, a pharmaceutically acceptable carrier may comprise one or more non-active pharmaceutical components. Non-active pharmaceutical components among the pharmaceutically acceptable carrier include stabilizers, preservatives, additives, adjuvants, sprays, other non-active pharmaceutical components suitable for use with pharmaceutically effective compounds, compressed air, or other suitable gases.

In a preferred embodiment, the pharmaceutically acceptable solvent is selected from the group consisting of sterile water, decarbonated water, ethanol, aqueous ethanol solution, aqueous sorbitol solution, normal saline, and combinations thereof.

The inventor mixed the commonly used pharmaceutically acceptable solvent, including sterile water, decarbonated water, ethanol, aqueous ethanol solution, aqueous sorbitol solution, and normal saline, with Kuding saponin compounds respectively; and the result showed that the aqueous ethanol solution is optimal in solubility with above-mentioned Kuding saponin compound, and a stable solution or suspension can be formed thereof.

Therefore, in a preferred embodiment, the pharmaceutically acceptable solvent is aqueous ethanol solution.

In a more preferred embodiment, among the pharmaceutically acceptable solvent, the mass percentage of ethanol in the whole liquid pharmaceutical composition is 1.0-5.0%; more preferably 2.5-4.0%, and even more preferably 3.5%.

"Pharmaceutically acceptable cosolvent" refers to compounds added into the solvent, which form a complex, a conjugate or a complex salt between soluble molecules with a poorly soluble medicament in order to increase the solubility of a drug, and normally do not substantially reduce the efficacy of the medicament.

In a preferred embodiment, the pharmaceutically acceptable cosolvent is selected from the group consisting of propanediol, glycerol, polyethylene glycol 200, polyethylene glycol 400 or Tween 80; preferably Tween 80. Normally, based on the selected cosolvent type, the mass percentage of the cosolvent in the whole liquid pharmaceutical composition is 0.1% -1.0%, for example, 0.1-0.8% or 0.1% -0.5%.

In a preferred embodiment, the cosolvent is Tween 80, and the mass percentage thereof in the whole liquid pharmaceutical composition is 0.1% -0.5%, preferably 0.2%-0.4%, more preferably 0.3%.

"Pharmaceutically acceptable stabilizers" refer to compounds capable of increasing the stability of solutions, colloids, solids, and mixtures, which can achieve the stabilizing effect by slowing down reactions, maintaining chemical equilibrium, reducing surface tension, preventing light and/or thermal decomposition or oxidative decomposition, and normally do not substantially reduce the therapeutical effect of the medicament.

In a preferred embodiment, the pharmaceutically acceptable stabilizer is selected from phenylethanol or disodium edetate, preferably disodium edetate. In general, depending on the type of stabilizer selected, the mass percentage of the stabilizer in the whole liquid pharmaceutical composition is 0.01%-0.50%, such as 0.05% -0.20%.

In a preferred embodiment, the stabilizer is disodium edetate, and the mass percentage thereof in the whole liquid pharmaceutical composition is 0.05% -0.20%, preferably 0.05% - 0.10%, more preferably 0.072% -0.088%.

As used herein, the term "buffer" is well known to those skilled in the art and can be safely used in pharmaceutical formulations, including but not limited to glycine-hydrochloric acid buffer, phthalic acid-hydrochloric acid buffer, disodium hydrogen phosphate-citric acid buffer, citric acid-sodium hydroxide-hydrochloric acid buffer, citric acid-sodium citrate buffer, acetic acid-sodium acetate buffer, disodium hydrogen phosphate-sodium dihydrogen phosphate buffer, disodium hydrogen phosphate-potassium dihydrogen phosphate buffer, potassium dihydrogen phosphate-sodium hydroxide buffer, sodium barbital-hydrochloric acid buffer, Tris-hydrochloric acid buffer, boric acid-borax buffer, glycine-sodium hydroxide buffer, borax-sodium hydroxide buffer, sodium carbonate-sodium bicarbonate buffer, PBS buffer (disodium hydrogen phosphate-sodium dihydrogen phosphate-sodium chloride buffer), and etc. The above buffers are commercially available to those skilled in the art, or those skilled in the art can formulate a buffer with a certain pH value according to the needs. Unless otherwise stated, all the "pH value" in the present invention is the pH value measured at 25° C.

In the liquid pharmaceutical composition of the present invention, it is preferred to use a buffer having a pH of 6.5-7.5. In a preferred embodiment, the buffer of pH 6.5-7.5 is selected from the group consisting of a phosphate buffer, preferably a sodium phosphate buffer, a potassium phosphate buffer, or a combination thereof, more preferably a disodium hydrogen phosphate-potassium dihydrogen phosphate buffer, most preferably a disodium hydrogen phosphate-potassium dihydrogen phosphate buffer having a pH of 6.5, a disodium hydrogen phosphate-potassium dihydrogen phosphate buffer having a pH of 6.9, a disodium hydrogen phosphate-potassium dihydrogen phosphate buffer having a pH of 7.0, a disodium hydrogen phosphate-potassium dihydrogen phosphate buffer having a pH of 7.4, or a disodium hydrogen phosphate-potassium dihydrogen phosphate buffer having a pH of 7.5.

The term "buffering effective amount" means that the concentration of the buffer is sufficient to maintain the pH of the liquid pharmaceutical composition at a specific pH value (preferably pH 6.5-7.5) ± 0.5(preferably ± 0.3, more preferably ± 0.2, most preferably ± 0.1) pH units. For example but without limitation, pH 6.5 ± 0.2(i.e. pH 6.3-6.7), pH 6.5 ± 0.3(i.e. pH 6.2-6.8), and etc. The specific concentration of the buffer is related to factors such as the type of the buffer solution, pH value, the particular composition of the whole pharmaceutical composition, and the like, which can be conveniently determined by those skilled in the art within a limited number of routine experiments. In general, the buffer has a concentration of 5mM to 500mM (preferably 5 mM to 100 mM, more preferably 5 mM to 50 mM, and even more preferably 5 mM to 20 mM).

In a preferred embodiment, the buffer system of the liquid pharmaceutical composition is disodium hydrogen phosphate-dipotassium hydrogen phosphate, and the pH of the composition is about 7.0; wherein, based on the total weight of the liquid pharmaceutical composition, the liquid pharmaceutical composition comprises: 0.006% -0.030% of Kudinoside A, about 3.5% of ethanol, 0.1% -0.5% of Tween 80, and 0.072%-0.088% of disodium edetate.

In a preferred embodiment, the liquid pharmaceutical composition is characterized in that the liquid pharmaceutical composition comprises, based on the total weight of the liquid pharmaceutical composition, about 0.006% of Kudinoside A, about 3.5% of ethanol, about 0.1% of Tween 80, about 0.072% of disodium edetate, and about 96.2% of water; or about 0.015% of Kudinoside A, about 3.5% of ethanol, about 0.3% of Tween 80, about 0.08% of disodium edetate, and about 95.8% of water; or about 0.03% of Kudinoside A, about 3.5% of ethanol, about 0.5% of Tween 80, about 0.088% of disodium edetate, and about 95.2% of water.

According to the disclosure of CN106456657A, the liquid pharmaceutical composition of the present invention can dilate respiratory tract smooth muscle and is suitable for treating diseases such as COPD and asthma. Inhalation therapy is the most common method currently recommended to treat asthma, and the dosage forms adopted for inhalation therapy include inhalation aerosol, dry powder inhalant, atomized inhalant, inhalation solution or suspension, and the like.

And therefore, in a preferred embodiment, the liquid pharmaceutical composition is a solution or a suspension.

In a more preferred embodiment, the dosage form of the liquid pharmaceutical composition is an inhalation solution or an inhalation suspension. In a specific embodiment of the present invention, the long-term stability test is mainly to investigate the targets such as the content of the ethanol and the content of the Kuding saponin compound. Assuming that the ethanol content in the liquid pharmaceutical composition is 100%, then using the same detection means, if the ethanol content thereof changed to 70% or more (preferably 80% or more, and more preferably more than 90%) of the initial content under certain conditions for a period of time and the Kuding saponin compound content also changed to 90% -110% (preferably 95% -105%, more preferably 98% -102%) of the initial content under certain conditions for a period of time, it is deemed that the liquid pharmaceutical composition is "stable", which is also reliable for the treatment of asthma, COPD, or other related diseases.

In some preferred embodiments, the Kuding saponin compounds in the liquid pharmaceutical composition of the present invention is Kudinoside A, and the liquid pharmaceutical composition has one or more of the following features:
(1) After testing for 9 months under acceleration test conditions with a temperature of 40°C ± 2°C and a humidity ≤ 25%, the content of Kudinoside A is ≥95%, the content of Kudinoside D as impurities is ≤0.30%, the content of Kudinoside E as impurities is ≤0.30%, and the sum of impurities does not exceed 1.0%;
(2) After testing for 9 months under long-term test conditions with a temperature of 30°C ± 2°C and a humidity of 35% ± 5%, the content of Kudinoside A is ≥ 100%, the content of Kudinoside D as impurities is ≤ 0.10%, the content of Kudinoside E as impurities is ≤0.10%, and the sum of impurities does not exceed 0.50%;
(3) After testing for 9 months under long-term test conditions with a temperature of 25°C ± 2°C and a humidity of 40% ± 5%, the content of Kudinoside A is ≥ 100%, the content of Kudinoside D as impurities is ≤0.10%, the content of Kudinoside E as impurities is ≤0.05%, and the sum of impurities does not exceed 0.30%.

In some preferred embodiments, the Kuding saponin compounds in the liquid pharmaceutical composition of the present invention is Kudinoside A, and the liquid pharmaceutical composition has one or more of the following features:
(1) During the test for 9 months under an acceleration test conditions with a temperature of 40°C ± 2°C and a humidity ≤ 25%, the content of the Kudinoside A is 98% -106%, the content of the Kudinoside D as impurities is ≤ 0.30%, the content of the Kudinoside E as impurities is ≤ 0.30%, and the sum of the impurities does not exceed 1.0%;
(2) During the test for 18 months under long-term test conditions with a temperature of 30°C ± 2°C and a humidity of 35% ± 5%, the content of Kudinoside A is 99% -106%, the content of Kudinoside D as impurities is ≤0.20%, the content of Kudinoside E as impurities is ≤0.15%, and the sum of impurities does not exceed 0.60%;
(3) During the test for 18 months under long-term test conditions with a temperature of 25°C ± 2°C and a humidity of 40% ± 5%, the content of Kudinoside A is 99% -106%, the content of Kudinoside D as impurities is ≤0.15%, the content of Kudinoside E as impurities is ≤0.02%, and the sum of impurities does not exceed 0.40%.

In a preferred embodiment, the liquid pharmaceutical composition of the present invention is stored in a sealed pharmaceutically acceptable packaging material.

The term "pharmaceutically acceptable packaging material" means a packaging material, wherein there is no severe mutual impact between the container sealing material and the contents therein; and it does not cause changes in product activity and stability or cause a risk of toxicity; and further any influence generated between the packaging material composition and the product under normal storage/use conditions does not result in unacceptable changes to the product quality or packaging. The pharmaceutically acceptable packaging material comprises a polymer material, a glass bottle, an aluminum foil material, or a combination thereof.

In a preferred embodiment, the polymer material includes, but is not limited to, a low-density polyethylene film, a low-density polyethylene bag, a low-density polyethylene bottle, a high-density polyethylene film, a high-density polyethylene bottle, a polypropylene bottle, a polyethylene terephthalate bottle, a polyester/aluminum/polyethylene composite film, a polyester/aluminum/polyethylene composite bag, aluminum foil bag, glass bottle, or a combination thereof; the aluminum foil material comprises aluminum foil bag, preferably a medicinal aluminum foil bag.

It has been found that in a long-term storage (e.g., 180 days or even longer at room temperature), if the volatile amount of ethanol in the liquid pharmaceutical composition of the present invention (i.e., the ratio of volatile ethanol content to its original content) can be controlled within the range of 30% or less, preferably 20% or less, more preferably 10% or less, a stable liquid pharmaceutical composition can be provided.

And therefore, in a preferred embodiment, the pharmaceutically acceptable packaging material is a combination of a low-density polyethylene bottle and an aluminum foil bag, which can more effectively prevent the volatilization of ethanol.

In a more preferred embodiment, the liquid pharmaceutical composition is packaged with a low-density polyethylene bottle, preferably a low-density polyethylene bottle having a thickness of 0.5-2.0 mm, more preferably a low-density polyethylene bottle having a thickness of 0.8-1.2 mm, most preferably a low-density polyethylene bottle having a thickness of 1.0-1.2 mm; and then placed the bottle in an aluminum foil bag. It should be noted that, in order to reduce the volatilization of solvent for dissolving the liquid pharmaceutical composition after contacting air, thereby causing the precipitation of the medicament, a vacuum or near vacuum state shall exist between the polymer material and the aluminum foil material. The near vacuum state described herein generally refers to a pressure ≤50 kPa, preferably a pressure ≤20 kPa, more preferably a pressure ≤10 kPa, and most preferred a pressure ≤1 kPa.

The method for preparing the liquid pharmaceutical composition of the present invention comprises: preparing a buffer solution (such as disodium hydrogen phosphate-potassium dihydrogen phosphate buffer) with a stabilizer (for example, disodium edetate) dissolved therein, dissolving all solvents (for example, an aqueous ethanol solution) and cosolvents (for example, Tween 80) in the buffer solution, adding Kuding saponin compounds (for example, Kudinoside A), and stirring until the complete dissolution; or, first adding a solvent (such as an ethanol aqueous solution), a stabilizer (such as disodium edetate) and a cosolvent (for example, Tween 80) and a part of a buffer (such as disodium hydrogen phosphate-potassium dihydrogen phosphate buffer), so that the mass percentage of ethanol within the obtained mixed solution in the whole liquid pharmaceutical composition is less than 100% (preferably 50%), then adding Kuding saponin compounds (for example, Kudinoside A), and stirring until the complete dissolution, and then adding the remaining buffer solution (such as disodium hydrogen phosphate-potassium dihydrogen phosphate buffer); or, the method comprises: preparing respectively a buffer solution (for example, disodium hydrogen phosphate-potassium dihydrogen phosphate buffer solution) with a stabilizer (for example, disodium edetate) dissolved therein, and a mother liquor of Kuding saponin compounds (such as kudinoside A) dissolved in solvent (such as an ethanol aqueous solution) and cosolvent (such as Tween 80), mixing the buffer solution and the mother liquor, so as to prepare the liquid pharmaceutical composition of the present invention.

The present invention will be described in further detail below in connection with embodiments. It should be understood, however, that the examples are illustrated for the purposes of illustration only and are not intended to limit the scope of the invention.

The compound used in the embodiments of the present invention is Kudinoside A, i.e., 3β-12α-19 α-trihydroxy-ursane-13(18)-ene-28,20β-lactone-3-O-[β-D-glucosyl-(1→3)-[α-L-rhamnosyl-(1→2)]-α-L-arabinoside (hereinafter referred to as "KA"), which is commercially available, and the preparation method thereof is also well known to those skilled in the art, for example, reference can be made to CN 106456657A and "Triterpenes and triterpenoid saponins from the leaves of Ilex kudincha".

### Example 1: Preparation of KA Formulation

### 1. Solution Formulation

According to the formulations in Table 1, weighing a formulation amount of water for injection use, adding a formulation amount of disodium hydrogen phosphate, potassium dihydrogen phosphate, and disodium edetate, respectively, stirring uniformly, adjusting the pH to about 7, and preparing a buffer solution; meanwhile, weighing a formulation amount of ethanol and Tween 80, adding a part of the prepared buffer solution therein, stirring uniformly, formulating a solution with the ethanol concentration of about 50%, adding a formulation amount of Kudinoside A into the solution, and stirring until the complete dissolution; and finally, adding the rest of the buffer solution, and stirring uniformly. Three(3) formulations were obtained.

**Table 1: Formulations**

| Material | Formulation 1 weight(g) | Formulation 2 weight(g) | Formulation 3 weight(g) |
|---|---|---|---|
| KA (pharmaceutical grade) | 1.80 (0.006%) | 4.50 (0.015%) | 9.00 (0.030%) |
| Absolute ethanol (pharmaceutical grade) | 1050.00 (3.50%) | 1050.00 (3.50%) | 1050.00 (3.50%) |
| Tween-80 (injection grade) | 30.00 (0.10%) | 90.00 (0.30%) | 150.00 (0.50%) |
| Disodium hydrogen phosphate (pharmaceutical grade) | 42.64 (0.14%) | 75.24 (0.25%) | 193.74 (0.65%) |
| Potassium dihydrogen phosphate (pharmaceutical grade) | 4.20 (0.014%) | 5.76 (0.0192%) | 8.00 (0.027%) |
| disodium edetate (pharmaceutical grade) | 21.60 (0.072%) | 24.00 (0.08%) | 26.40 (0.088%) |
| Water for injection | 28849.76 (96.17%) | 28750.50 (95.84%) | 28562.86 (95.21%) |
| Total amount | 30000.00 | 30000.00 | 30000.00 |

### 2. Filling

Low-density polyethylene (LDPE) bottles with three(3) different thicknesses (0.6 mm, 1.0 mm, 1.2 mm) were respectively filled with three(3) formulations by blowing, filling and sealing (BFS) integrated production line, and each bottle has 2 ml of the formulation.

### 3. Packaging

Package 1: Without any processing to the filled sample.
Package 2: The filled sample was packaged using an aluminum foil bag, and sealed.
Package 3: The filled sample was packaged using an aluminum foil bag, and vacuumized between the low-density polyethylene bottle and the aluminum foil bag to a pressure of ≤20 kPa.

The following nine(9) samples were prepared, specifically shown in Table 2 as Sample 1-Sample 9:

**Table 2: Sample Packaging**

| Low-density polyethylene bottle thickness (mm) | Package 1 | Package 2 | Package 3 |
|---|---|---|---|
| | No processing (bottle) | Alum foil bag, sealed (bottle) | Alum foil bag, vacuumed (bottle) |
| 0.6 | (Sample 1) Formulation 1 | (Sample 2) Formulation 2 | (Sample 3) Formulation 3 |
| 1.0 | (Sample 4) Formulation 3 | (Sample 5) Formulation 1 | (Sample 6) Formulation 2 |
| 1.2 | (Sample 7) Formulation 2 | (Sample 8) Formulation 3 | (Sample 9) Formulation 1 |

### Example 2: Detection of Ethanol Content Stability in KA Formulations

Low-density polyethylene bottle (LDPE bottle) produced by BFS method is now used as the packaging material directly contacting the medicament for inhalation formulations. Compared with the previously used glass ampoule, the LDPE bottle has the advantages such as purity of particle material, fewer additives, easy to open the bottle in the later period, and etc. However, the semi-permeability of the LDPE bottle may result in a decrease of the content of certain solvents (e.g, ethanol) stored therein due to volatilization. Due to the decrease in the ethanol content, the solubility of the active compound KA decreases consequently, and therefore resulting in KA precipitation. Thus, in order to enhance the stability of the formulation and prolong the storage time thereof, the inventor needs to prevent ethanol volatilization. To this end, the inventor have explored the stability of ethanol content in different sample formulations in Table 2.

A stability test of ethanol content was performed on the nine (9) KA formulation samples obtained in Table 2, Example 1, and the changes of the ethanol content in a formulation under long-term test, acceleration test, and high-temperature test conditions were explored (see Table 3 for details).

**Table 3: Condition of Ethanol Content Stability Test**

| Item | Test condition |
|---|---|
| Long-term test | Temperature: 25°C±2°C; Humidity: 40%±5% |
| Acceleration test | Temperature: 40°C±2°C; Humidity: 22%±5% |
| High-temperature test | Temperature: 60°C±2°C |

The ethanol content was determined by the quantitative analysis of the high-performance liquid chromatography, i.e., the external standard method, as recited in the Section "General Technology Requirements" of "Chinese Pharmacopoeia (Four Part), 2020 Editi*on*", wherein the conditional parameters used by the high-performance liquid chromatography are as follows:
(1) Gas Phase Condition:
   Instrument: a gas chromatograph that configured with an FID detector and an automatic headspace sampler;
   Chromatographic column: 30m × 0.53mm, film thickness of 3.0 µm, and the chromatographic column is a capillary column with (6%) cyanopropylphenyl-(94%)dimethylpolysiloxane as a stationary liquid;
   Column temperature: starting temperature of 40°C for 2 minutes, then heating to 65°C at a rate of 3°C per minute, and then heating to 200°C at a rate of 25°C per minute, and maintaining for 10 minutes;
   Carrier gas: Nitrogen; split ratio: 1:1;
   Sample inlet temperature: 200° C;
   FID detector temperature: 220° C.
(2) Headspace Sampling Conditions:
   Thermostatic Furnace Temperature: 85° C;
   Sample Bottle Thermostatic Temperature Time: 20 minutes.

The measured ethanol content is shown in Tables 4-6 and FIG. 1A -1C, 2A -2C, 3A -3C:

**Table 4: Detection Results of Ethanol Content in Long-Term Tests**

| Sample | Ethanol content (%) | | | | | | | | | | |
|---|---|---|---|---|---|---|---|---|---|---|---|
| | D0 | D3 | D6 | D9 | D15 | D30 | D60 | D90 | D 120 | D150 | D180 |
| 1 | 3.51 | 3.47 | 3.52 | 3.45 | 3.42 | 3.44 | 3.43 | 3.26 | 3.02 | 2.65 | 2.23 |
| 2 | 3.51 | 3.49 | 3.50 | 3.44 | 3.30 | 3.46 | 3.50 | 3.30 | 3.48 | 3.45 | 3.46 |
| 3 | 3.51 | 3.48 | 3.49 | 3.48 | 3.46 | 3.44 | 3.49 | 3.38 | 3.49 | 3.50 | 3.48 |
| 4 | 3.55 | 3.46 | 3.52 | 3.47 | 3.43 | 3.47 | 3.46 | 3.30 | 3.12 | 2.80 | 2.48 |
| 5 | 3.55 | 3.46 | 3.50 | 3.44 | 3.48 | 3.43 | 3.50 | 3.35 | 3.42 | 3.45 | 3.44 |
| 6 | 3.55 | 3.44 | 3.51 | 3.47 | 3.45 | 3.42 | 3.51 | 3.40 | 3.46 | 3.45 | 3.47 |
| 7 | 3.51 | 3.50 | 3.50 | 3.45 | 3.49 | 3.49 | 3.51 | 3.41 | 3.18 | 2.91 | 2.49 |
| 8 | 3.51 | 3.49 | 3.49 | 3.49 | 3.47 | 3.48 | 3.50 | 3.37 | 3.42 | 3.46 | 3.45 |
| 9 | 3.51 | 3.48 | 3.46 | 3.47 | 3.49 | 3.43 | 3.52 | 3.46 | 3.48 | 3.48 | 3.46 |

**Table 5: Detection Results of Ethanol Content in the Acceleration Test**

| Sample | Ethanol content (%) | | | | | | | | | | |
|---|---|---|---|---|---|---|---|---|---|---|---|
| | D0 | D3 | D6 | D9 | D15 | D30 | D60 | D90 | D 120 | D150 | D180 |
| 1 | 3.51 | 3.47 | 3.33 | 3.48 | 3.36 | 3.36 | 2.90 | 2.56 | 2.14 | 1.91 | 1.88 |
| 2 | 3.51 | 3.48 | 3.46 | 3.46 | 3.44 | 3.48 | 3.49 | 3.31 | 3.22 | 3.12 | 3.02 |
| 3 | 3.51 | 3.46 | 3.47 | 3.47 | 3.45 | 3.47 | 3.51 | 3.45 | 3.34 | 3.30 | 3.22 |
| 4 | 3.55 | 3.41 | 3.45 | 3.44 | 3.40 | 3.37 | 3.25 | 2.98 | 2.45 | 2.23 | 2.19 |
| 5 | 3.55 | 3.45 | 3.47 | 3.47 | 3.46 | 3.49 | 3.46 | 3.36 | 3.31 | 3.22 | 3.12 |
| 6 | 3.55 | 3.44 | 3.47 | 3.47 | 3.41 | 3.48 | 3.47 | 3.40 | 3.39 | 3.41 | 3.42 |
| 7 | 3.51 | 3.49 | 3.47 | 3.50 | 3.43 | 3.45 | 3.32 | 3.22 | 2.96 | 2.52 | 2.45 |
| 8 | 3.51 | 3.46 | 3.48 | 3.48 | 3.45 | 3.49 | 3.48 | 3.38 | 3.35 | 3.30 | 3.32 |
| 9 | 3.51 | 3.48 | 3.47 | 3.47 | 3.47 | 3.51 | 3.49 | 3.46 | 3.41 | 3.43 | 3.40 |

**Table 6: Detection Results of Ethanol Content in the High-Temperature Tests**

| Sample | Ethanol content (%) | | | | | | | | | | |
|---|---|---|---|---|---|---|---|---|---|---|---|
| | D0 | D3 | D6 | D9 | D15 | D30 | D60 | D90 | D 120 | D 150 | D180 |
| 1 | 3.51 | 3.29 | 3.25 | 3.01 | 2.96 | 2.36 | 1.18 | 1.20 | 1.01 | 0.98 | 0.91 |
| 2 | 3.51 | 3.42 | 3.44 | 3.45 | 3.32 | 3.36 | 2.92 | 2.45 | 2.40 | 2.28 | 2.19 |
| 3 | 3.51 | 3.48 | 3.48 | 3.49 | 3.44 | 3.40 | 3.36 | 3.20 | 2.85 | 2.50 | 2.32 |
| 4 | 3.55 | 3.43 | 3.31 | 3.21 | 2.99 | 2.45 | 1.62 | 1.35 | 1.23 | 1.06 | 0.99 |
| 5 | 3.55 | 3.41 | 3.41 | 3.41 | 3.40 | 3.25 | 2.96 | 2.48 | 2.36 | 2.25 | 2.20 |
| 6 | 3.55 | 3.44 | 3.42 | 3.46 | 3.44 | 3.42 | 3.44 | 3.41 | 3.35 | 3.33 | 3.32 |
| 7 | 3.51 | 3.42 | 3.35 | 3.27 | 3.14 | 2.55 | 2.15 | 2.01 | 1.86 | 1.75 | 1.65 |
| 8 | 3.51 | 3.44 | 3.36 | 3.44 | 3.38 | 3.25 | 3.10 | 3.05 | 2.77 | 2.32 | 2.26 |
| 9 | 3.51 | 3.46 | 3.43 | 3.48 | 3.41 | 3.46 | 3.42 | 3.36 | 3.31 | 3.25 | 3.23 |

As shown in Tables 4-6 and FIGS. 1A -1C, 2A -2C and 3A -3C, it can be seen that under long-term test, acceleration test, and high-temperature test conditions, the ethanol contents of Samples 1, 4, and 7 were significantly reduced, which indicates that if the polyethylene bottle storing the sample is not subjected to any package processes, the ethanol content of the stored sample therein will continuously reduce over time; and, regardless of any stability test condition, the ethanol contents of Sample 6 and Sample 9 are substantially maintained as stable, which indicates that using a low-density polyethylene material with a certain thickness (e.g., more than 0.6 mm or preferably more than 1.0 mm), packaging with an aluminum foil bag, and vacuumizing between the low-density polyethylene bottle and the bag will be most favorable for maintaining the ethanol content.

On the other hand, it was observed that under long-term test conditions, Samples 1, 4, 7 began precipitation at day 180; under acceleration test conditions, Samples 1, 4, and 7 began precipitation at day 90, 120, and 150, respectively; and under high-temperature test conditions, Samples 1, 4, and 7 all began precipitation at day 30, Sample 2 began precipitation at day 90, Sample 5 began precipitation at day 120, Sample 3 began precipitation at day 150, and Sample 8 began precipitation at day 180, in contrast, Samples 6 and 9 did not precipitate all the time. As illustrated, in parts of the samples, KA started to precipitate with the decrease of the ethanol content.

In summary, using a composite package of a low-density polyethylene bottle with a certain thickness (e.g., more than 0.6 mm, preferably more than 1.0) and an aluminum foil bag, and when there is a vacuum or near vacuum state exists between these two, the ethanol content in the stored sample can substantively maintain stable, and there is no precipitation of Kudinoside A, thus it is favorable for sample storage.

### Example 3: Stability Test of KA Formulations

The sample 6 shown in Example 1 was selected, and a stability test was performed according to the conditions shown in Table 7:

**Table 7: Stability Test Conditions**

| Item | Test condition |
|---|---|
| Acceleration test | Temperature: 40°C±2°C; Humidity: ≤25% |
| Long-term test 1 | Temperature: 30°C±2°C; Humidity: 35%±5% |
| Long-term test 2 | Temperature: 25°C±2°C; Humidity: 40%±5% |

The KA content was determined by the external standard method of the high-performance liquid chromatography as recited in the Section 0512 "General Technology Requirements" of "Chinese Pharmacopoeia (Four Part), 2020 Editi*on*", wherein the chromatographic conditional parameters used by the high-performance liquid chromatography are as follows:
Chromatographic column: C18(2.1^{∗}100mm, 1.8µm);
Mobile Phase: Acetonitrile: Water (26: 74);
Flow rate: 0.6 mL/min;
Column temperature: 38° C;
Detection wavelength: 225 nm;
Loading amount: 5 µL;
Elution Time: 28 min.

The relevant substance contents in the formulations were determined by the self-control method of main components plus correction factor of the high-performance liquid chromatography as recited in the Section 0512 "General Technology Requirements" of "Chinese Pharmacopoeia (Four Part), 2020 Editi*on",* wherein the chromatographic conditional parameters used by the ultra-high pressure liquid chromatography are as follows:
Chromatographic column: C18(2.1^{∗}100mm, 1.8µm);
Mobile Phase: Phase A: water, and phase B: Acetonitrile;
Column temperature: 50° C;
Detection wavelength: 225 nm;
Loading amount: 5 µL;

The gradient elution conditions are as follows:

| Time [min] | Mobile phaseA [water%V/V] | Mobile phase B [Acetonitrile %V/V] | Flow rate [mL/min] | Max. pressure limit [bar] |
|---|---|---|---|---|
| 0 | 77 | 23 | 0.600 | 1200.00 |
| 30 | 70 | 30 | 0.600 | 1200.00 |
| 35 | 70 | 30 | 0.600 | 1200.00 |
| 36 | 77 | 23 | 0.600 | 1200.00 |
| 40 | 77 | 23 | 0.600 | 1200.00 |

The results of stability tests under three(3) different conditions are shown in Table 8-10 as follows:

**Table 8: Acceleration Test**

| Investigation Item | Quality standard | Time (month) | | | | | |
|---|---|---|---|---|---|---|---|
| | | 0 | 1 | 2 | 3 | 6 | 9 |
| KA content (%) | The primary medicament KA, shall be 90%-110% of the labeled amount | 99.20 | 104.1 | 104.4 | 104.6 | 100.7 | 98.9 |
| Relevant substances (%) | Impurity F (Kudinoside D), shall not exceed 1.5% of the labeled amount of the primary medicament KA | 0.09 | 0.08 | 0.1 | 0.12 | 0.19 | 0.30 |
| | Impurity G (Kudinoside E), shall not exceed 1.0% of the labeled amount of the primary medicament KA | ND | ND | ND | 0.09 | 0.18 | 0.30 |
| | The sum of impurities, shall not exceed 5.0% of the labeled amount of the primary medicament KA | 0.09 | 0.08 | 0.2 | 0.60 | 0.67 | 0.83 |

| | | | | | | | |
|---|---|---|---|---|---|---|---|
| Note: ND refers to "not detected". | | | | | | | |

**Table 9: Long-Term Test 1**

| Investigation Item | Quality standard | Time (month) | | | | | | | |
|---|---|---|---|---|---|---|---|---|---|
| | | 0 | 1 | 2 | 3 | 6 | 9 | 12 | 18 |
| KA content (%) | The primary medicament KA, shall be 90%-110% of the labeled amount | 99.2 | 103.8 | 104.6 | 104.5 | 100.7 | 104.8 | 103.9 | 104.8 |
| Relevant substances (%) | Impurity F (Kudinoside D), shall not exceed 1.5% of the labeled amount of the | 0.09 | 0.06 | 0.06 | 0.08 | 0.09 | 0.10 | 0.12 | 0.16 |
| | primary medicament KA | | | | | | | | |
| | Impurity G (Kudinoside E), shall not exceed 1.0% of the labeled amount of primary medicament KA | ND | ND | ND | ND | ND | 0.03 | 0.03 | 0.12 |
| | The sum of impurities, shall not exceed 5.0% of the labeled amount of the primary medicament KA | 0.09 | 0.06 | 0.14 | 0.39 | 0.53 | 0.44 | 0.12 | 0.28 |

| | | | | | | | | | |
|---|---|---|---|---|---|---|---|---|---|
| Note: ND refers to "not detected". | | | | | | | | | |

**Table 10: Long-Term Test 2**

| Investigation Item | Quality standard | Time (month) | | | | | | | |
|---|---|---|---|---|---|---|---|---|---|
| | | 0 | 1 | 2 | 3 | 6 | 9 | 12 | 18 |
| KA content (%) | The primary medicament KA, shall be 90%-110% of labeled amount | 99.2 | 104.4 | 105.5 | 104.9 | 101.8 | 103.3 | 104.7 | 103.8 |
| Relevant substances (%) | Impurity F (Kudinoside D), shall not exceed 1.5% of the labeled amount of the primary medicament KA | 0.09 | 0.06 | 0.06 | 0.08 | 0.07 | 0.09 | 0.09 | 0.12 |
| | Impurity G (Kudinoside E), shall not exceed 1.0% of the labeled amount of the primary medicament KA | ND | ND | ND | ND | ND | 0.02 | ND | ND |
| | The sum of impurities, shall not exceed 5.0% of the labeled amount of the primary medicament KA | 0.09 | 0.06 | 0.06 | 0.24 | 0.37 | 0.30 | 0.09 | 0.21 |

| | | | | | | | | | |
|---|---|---|---|---|---|---|---|---|---|
| Note: ND refers to "not detected". | | | | | | | | | |

As can be seen from the results shown in Table 8-10, regardless of under any test conditions, the KA formulation prepared in Example 1 Sample 6, conforms to the specified quality standards, indicating a good stability.

Although the present invention describes specific examples, it will be apparent to those skilled in the art that a variety of changes and modifications can be made to the present invention without departing from the spirit and scope of the invention. Accordingly, the appended claims cover all such variations within the scope of the invention.

## Claims

1. A stable liquid pharmaceutical composition, wherein the liquid pharmaceutical composition comprises a therapeutically effective amount of a Kuding saponin compound, a buffering effective amount of a buffer solution having a pH of 6.5-7.5, and a pharmaceutically acceptable carrier.

2. The liquid pharmaceutical composition according to claim 1, wherein the Kuding saponin compound is a compound of formula (I): 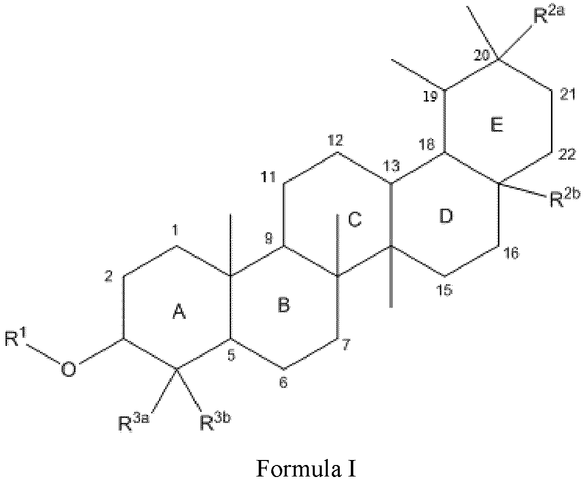 wherein:
ring A, ring B, ring C, ring D, or ring E are each independently a fully saturated or partially saturated ring;
positions C2, C11, C12, and C19 are independently optionally substituted by-OH, respectively;
R¹ is a sugar residue, preferably a monosaccharide residue or an oligosaccharide residue;
R^{2a} and R^{2b} together form -CO₂-;
R^{3a} and R^{3b} together form CH₂=, or are independently selected from -CH₃ or -CH₂-OH, respectively.

3. The liquid pharmaceutical composition according to claim 2, wherein:
in the formula (I), the ring A, ring B, ring C, and ring E are fully saturated rings, the ring D is a partially saturated ring, positions C12 and C19 are independently substituted by -OH, respectively, R¹ is a monosaccharide residue or an oligosaccharide residue, and R^{3a} and R^{3b} are - CH₃, respectively; or
in formula (I), the ring A, ring B, ring C, and ring E are fully saturated rings, ring D is a partially saturated ring, positions C11 and C19 are independently substituted by -OH, respectively, R^{3a} and R^{3b} are -CH₃, respectively, and R¹ is a monosaccharide residue or an oligosaccharide residue; or
in formula (I), ring A, ring B, and ring E are fully saturated rings, ring C and ring D are partially saturated rings, position C19 is substituted by -OH, R^{3a} and R^{3b} are -CH₃, respectively, and R¹ is a monosaccharide residue or an oligosaccharide residue;
preferably, the compound of formula (I) has a structure of following formula (II), (III), or (IV): 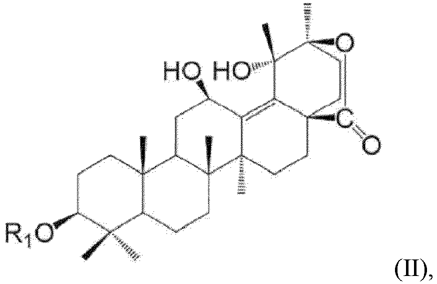 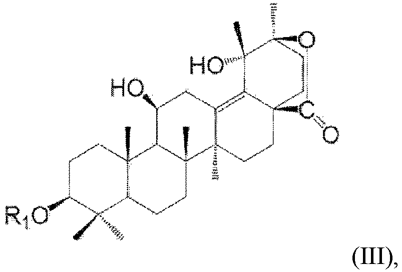 or 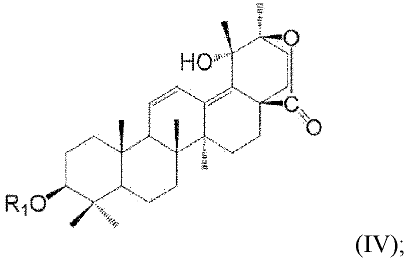 in formulae (II), (III) and (IV), R₁ is a monosaccharide residue or an oligosaccharide residue; preferably, the monosaccharide is arabinose, glucuronic acid, 2-deoxy-glucuronic acid, glucose or rhamnose; the oligosaccharide residue is a disaccharide residue, a trisaccharide residue, or a tetrasaccharide residue; preferably, the oligosaccharide residues comprise any combination of glucose, arabinose, and rhamnose.

4. The liquid pharmaceutical composition according to claim 1, wherein the Kuding saponin compound is selected from the group consisting of Kudinoside A, Kudinoside B, Kudinoside C, Kudinoside D, Kudinoside E, Kudinoside F, Kudinoside I, Kudinoside J, Ilekudinoside H, Ilekudinoside I, and Ilekudinoside J; preferably, the Kuding saponin compound is selected from the group consisting of Kudinoside A, Kudinoside B, Kudinoside C, Kudinoside D, Kudinoside I, Ilekudinoside I, and Ilekudinoside J; and more preferably, the Kuding saponin compound is 3β-12α-19α-trihydroxy-ursane-13(18)-ene-28, 20β-lactone-3-O-[β-D-glucosyl-(1→3)-[α-L-rhamnosyl-(1→2)]-α-L-arabinoside and/or 3β-12β-19α-trihydroxy-ursane-13(18)-ene-28,20β-lactone-3-O-[P-D-glucosyl-(1→3)-[α-L-rhamnosyl-(1 → 2) ]-α-L-arabinoside.

5. The liquid pharmaceutical composition according to claim 1, wherein the Kuding saponin compound is separated from a plant of genus *Ilex* in family *Aquifoliaceae*;
preferably, the plant of genus *Ilex* in family *Aquifoliaceae* is selected from the group consisting of *Ilex kudingcha C.J Tseng, Ilex latifolia Thunb., Ilex cornute Lindl., Ilex. Pentagona S.K.Chen. Y.X.Feng et C.F.Liang, Ilex centro-chinensis S. Y.Hu* and *Ilex houshanensis Y.H.He.*

6. The liquid pharmaceutical composition according to claim 1, wherein the liquid pharmaceutical composition is a solution or a suspension, preferably a dosage form of an inhalation solution or an inhalation suspension.

7. The liquid pharmaceutical composition according to claim 1, wherein the mass percentage of Kuding saponin compound in the whole liquid pharmaceutical composition is 0.001-0.050%, preferably 0.006-0.030%, more preferably 0.010-0.030%, and even more preferably 0.015%.

8. The liquid pharmaceutical composition of claim 1, wherein the buffer solution having a pH of 6.5-7.5 is a phosphate buffer, preferably a sodium phosphate buffer, a potassium phosphate buffer, or a combination thereof; more preferably a disodium hydrogen phosphate-potassium dihydrogen phosphate buffer; even more preferably a disodium hydrogen phosphate-potassium dihydrogen phosphate buffer having a pH of 6.5, a disodium hydrogen phosphate-potassium dihydrogen phosphate buffer having a pH of 6.9, a disodium hydrogen phosphate-potassium dihydrogen phosphate buffer having a pH of 7.0, a disodium hydrogen phosphate-potassium dihydrogen phosphate buffer having a pH of 7.4, or a disodium hydrogen phosphate-potassium dihydrogen phosphate buffer having a pH of 7.5.

9. The liquid pharmaceutical composition according to claim 1, wherein the pharmaceutically acceptable carrier comprises a pharmaceutically acceptable solvent, a cosolvent, a stabilizer, or a combination thereof.

10. The liquid pharmaceutical composition according to claim 9, wherein the pharmaceutically acceptable solvent is selected from water, ethanol, or a combination thereof; preferably an aqueous ethanol solution; more preferably, among the pharmaceutically acceptable solvent, the mass percentage of ethanol in the whole liquid pharmaceutical composition is 1.0-5.0%; more preferably 2.5-4.0%, and most preferably 3.5%.

11. The liquid pharmaceutical composition according to claim 9, wherein the cosolvent is selected from the group consisting of propanediol, glycerol, polyethylene glycol 200, polyethylene glycol 400, or Tween 80; preferably Tween 80; preferably, the mass percentage of the cosolvent in the whole liquid pharmaceutical composition is 0.1% -0.8%, preferably 0.2% - 0.5%;
more preferably, the cosolvent is Tween 80, and the mass percentage of the Tween 80 in the whole liquid pharmaceutical composition is 0.1% -0.5%, more preferably 0.3%.

12. The liquid pharmaceutical composition according to claim 9, wherein the stabilizer is selected from the group consisting of phenylethanol or disodium edetate, preferably disodium edetate; preferably, the mass percentage content of the stabilizer in the whole liquid pharmaceutical composition is 0.05%-0.20%, preferably 0.05% -0.15%;
more preferably, the stabilizer is disodium edetate, and the mass percentage content of the disodium edetate in the whole liquid pharmaceutical composition is 0.05%-0.20%; more preferably, 0.05%-0.10%, most preferably 0.072% -0.088%.

13. A liquid pharmaceutical composition, wherein the buffer system of the liquid pharmaceutical composition is disodium hydrogen phosphate-potassium dihydrogen phosphate, and the pH of the composition is about 7.0; wherein the liquid pharmaceutical composition comprises, based on the whole weight of the liquid pharmaceutical composition:
0.006% -0.030% of Kudinoside A;
about 3.5% of ethanol;
0.1% -0.5% of Tween 80; and
0.072% -0.088% of disodium edetate.

14. The liquid pharmaceutical composition according to claim 13, wherein the liquid pharmaceutical composition comprises, based on the total weight of the liquid pharmaceutical composition: about 0.006% of Kudinoside A, about 3.5% of ethanol, about 0.1% of Tween 80, about 0.072% of disodium edetate, and about 96.2% of water; or about 0.015% of Kudinoside A, about 3.5% of ethanol, about 0.3% of Tween 80, about 0.08% of disodium edetate, and about 95.8% of water; or
about 0.03% Kudinoside A, about 3.5% of ethanol, about 0.5% of Tween 80, about 0.088% of disodium edetate, and about 95.2% of water.

15. A packaged pharmaceutical product, wherein the packaged pharmaceutical product comprises a sealable container formed by a pharmaceutically acceptable packaging material, wherein the sealable container comprises the liquid pharmaceutical composition according to any one of claims 1-14.

16. The packaged pharmaceutical product according to claim 15, wherein the pharmaceutically acceptable packaging material is selected from the group consisting of a polymeric material and an aluminum foil material or a combination thereof;
wherein the polymer material is selected from the group consisting of a low-density polyethylene film, a low-density polyethylene bag, a low-density polyethylene bottle, a high-density polyethylene film, a high-density polyethylene bottle, a polypropylene bottle, a polyethylene terephthalate bottle, a glass bottle, a polyester/aluminum/polyethylene composite film, a polyester/aluminum/polyethylene composite bag, or a combination thereof;
the aluminum foil material comprises an aluminum foil bag, preferably a medicinal aluminum foil bag;
preferably, the pharmaceutically acceptable packaging material is a combination of a low-density polyethylene bottle and an aluminum foil bag.

17. The packaged pharmaceutical product according to claim 16, wherein the liquid pharmaceutical composition is sealed within a sealable container formed by the polymer material and the aluminum foil material, and a vacuum or near vacuum state exists between the polymer material and the aluminum foil material.

18. The liquid pharmaceutical composition according to claim 16 or 17, wherein the low-density polyethylene bottle has a thickness of 0.5-2.0 mm, preferably 0.8-1.2 mm, more preferably 1.0-1.2 mm.
